(19) Europäisches Patentamt
European Patent Office
Office européen des brevets

(11) Veröffentlichungsnummer : **0 083 442**
**B1**

(12) # EUROPÄISCHE PATENTSCHRIFT

(45) Veröffentlichungstag der Patentschrift : **20.08.86**

(51) Int. Cl.[4] : **C 07 C 87/60**, C 07 C 85/24

(21) Anmeldenummer : **82111987.2**

(22) Anmeldetag : **24.12.82**

(54) **Verfahren zur Herstellung von 2,4,6-Trichloranilin.**

(30) Priorität : **05.01.82 DE 3200069**

(43) Veröffentlichungstag der Anmeldung : **13.07.83 Patentblatt 83/28**

(45) Bekanntmachung des Hinweises auf die Patenterteilung : **20.08.86 Patentblatt 86/34**

(84) Benannte Vertragsstaaten : **BE CH DE FR GB LI**

(56) Entgegenhaltungen :
**DE-A- 2 449 285**
**US-A- 2 675 409**
**BERICHTE DER DEUTSCHEN CHEMISCHEN GESELLSCHAFT, Band 55, 1922, Berlin; W. ELLER et al. "Über die Einwirkung von Sulfurylchlorid auf aromatische Amine", Seiten 217-224**

(73) Patentinhaber : **BAYER AG**
**Konzernverwaltung RP Patentabteilung**
**D-5090 Leverkusen 1 Bayerwerk (DE)**

(72) Erfinder : **Werner, Friedrich, Dr. c/o Bayer do Brasil S. A.**
**Estrada Boa Esperanza s/n**
**BR-26150 Belford Roxo (BR)**
Erfinder : **Mannes, Karl, Dr.**
**Kurt-Schumacher-Ring 119**
**D-5090 Leverkusen (DE)**
Erfinder : **Trescher, Viktor, Dr.**
**Völklinger Strasse 7**
**D-5090 Leverkusen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 083 442 B1

**Beschreibung**

Die Erfindung betrifft ein Verfahren zur Herstellung von 2,4,6-Trichloranilin durch Umsetzung von gegebenenfalls durch Halogen substituiertem Anilin und/oder dessen Hydrochlorid mit einem Chlorierungsmittel in Gegenwart von inerten, organischen Lösungs- und/oder Verdünnungsmitteln.

Es ist bekannt, 2,4,6-Trichloranilin durch Umsetzung von Anilin oder dessen Hydrochlorid mit Chlor oder Sulfurylchlorid in Gegenwart von inerten, organischen Lösungsmitteln herzustellen (vgl. z. B. Liebigs Ann. Chem. *53*, 35 (1845), J. prak. Chem. (2), *16*, 449 (1877), Liebigs Ann. Chem. *196*, 230 (1879), Ber. *27*, 3151 (1894), Ber. *55*, 221 (1922), Metody Peluchemia khim. Reaktinov preperatov *26*, 280-282 (1974) (zit. in CA *83*, 113802 v), US-PS 2 675 409). Bei den genannten Verfahren wird im allgemeinen auf die Temperaturführung besonderen Wert gelegt. So wird z. B. darauf geachtet, daß beim Zusammengeben der Reaktionspartner die Temperatur nicht allzu stark ansteigt, was durch Kühlung des Reaktionsgemisches bewerkstelligt werden kann (vgl. z. B. Liebigs Ann. Chem. *196*, 231 (1879)). Obwohl man üblicherweise das Reaktionsgemisch anschließend zur Vervollständigung der Reaktion auf etwas höhere Temperaturen erwärmt, wird ausdrücklich in Ber. *55*, 221 (1922) darauf hingewiesen, daß die Erwärmung des Reaktionsgemisches 70 °C nicht übersteigen soll. In der US-PS 2 675 409 wird zwar in Spalte 3, Zeilen 29 bis 33 erwähnt, daß die Temperatur für die Chlorierung ziemlich unwichtig ist, praktisch wird aber bei Temperaturen von 0 bis 40 °C gearbeitet.

Nachteilig bei den genannten Verfahren sind die für ein großtechnisches Verfahren unbefriedigenden Ausbeuten an 2,4,6-Trichloranilin, das zudem oftmals in beträchtlichem Maße durch Nebenprodukte verunreinigt ist, was eine kostenintensive Nachreinigung erforderlich macht.

In Ber. *55*, 221 (1922) wird zwar angegeben (s. Beispiel 2), daß reines 2,4,6-Trichloranilin ausgehend von Anilinhydrochlorid in einer Ausbeute von 90 bis 95 % der Theorie erhalten wird. Diese Angabe konnte aber bei einer neuerlichen Nacharbeitung des Verfahrens (vgl. Metody Peluchemia khim. Reaktinov preperatov *26*, 280-282 (1974)) nicht bestätigt werden. Bei der Nacharbeitung wurde lediglich eine Ausbeute von ca. 60 % an reinem 2,4,6-Trichloranilin erzielt. Auch die in dem Beispiel der US-PS 2 675 409 angegebene Ausbeute an 2,4,6-Trichloranilin von 92 % bezieht sich nur auf ein stark verunreinigtes Rohprodukt, dessen Gehalt an 2,4,6-Trichloranilin nur etwa 65 % beträgt, wie eigene Vergleichsversuche zeigen (s. Vergleichsbeispiel).

In der DE-OS 2 449 285 ist ein allgemeines Verfahren zur Chlorierung von Anilinen bei Temperaturen von 10 bis 80 °C beschrieben. Es wird aber ausdrücklich darauf hingewiesen (vgl. S. 4, 2. Abs. der DE-OS), daß ein größerer Anteil an höherchlorierten Produkten vermieden werden soll. Danach richten sich auch die dort angegebenen Molverhältnisse von eingesetztem Amin zu eingesetztem Chlor. Gemäß Beispiel 2 der genannten DE-OS erhält man bei der Chlorierung von Anilin in Chlorbenzol bei 27 bis 32 °C ein Produktgemisch, das nur in untergeordnetem Maße 2,4,6-Trichloranilin enthält.

Es wurde nun ein Verfahren zur Herstellung von 2,4,6-Trichloranilin durch Umsetzung von Anilinen der Formel

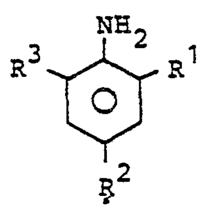

worin $R^1$, $R^2$ und $R^3$ gleich oder verschieden sind und für Wasserstoff oder Halogen stehen und wobei mindestens einer der Reste Wasserstoff bedeutet,
und/oder deren Hydrochloride mit einem Chlorierungsmittel in Gegenwart von inerten, organischen Lösungs- und/oder Verdünnungsmitteln gefunden, das dadurch gekennzeichnet ist, daß man die Umsetzung bei Temperaturen oberhalb von 80 °C durchführt.

Als Halogene seien genannt : Chlor, Brom, Jod, bevorzugt Chlor.

Als Ausgangsprodukte für das erfindungsgemäße Verfahren kommen beispielsweise in Frage : Anilin, 2-Chloranilin, 4-Chloranilin, 2,4-Dichloranilin oder 2,6-Dichloranilin, bevorzugt Anilin.

Das erfindungsgemäße Verfahren wird im allgemeinen bei Temperaturen von etwa 82 bis 180 °C, bevorzugt bei 85 bis 175 °C, besonders bevorzugt bei 90 bis 170 °C, durchgeführt.

Als Chlorierungsmittel können in das erfindungsgemäße Verfahren eingesetzt werden : Stickstofftrichlorid, N,N-Dichlorethylamin, N-2,4-trichloracetanilid, Sulfurylchlorid und/oder elementares Chlor, bevorzugt elementares Chlor und/oder Sulfurylchlorid.

Die Chlorierungsmittel werden im allgemeinen im geringen molaren Überschuß in das erfindungsgemäße Verfahren eingesetzt. Es ist jedoch auch möglich, das Chlorierungsmittel in äquivalenten Mengen, bezogen auf das Ausgangsprodukt, einzusetzen. Üblicherweise setzt man das Chlorierungsmittel in einem molaren Überschuß von etwa 1 bis 20 Mol-%, bevorzugt 5 bis 15 Mol-%, pro einzuführendem Chlor ein.

Als inerte organische Lösungs- und/oder Verdünnungsmittel können solche eingesetzt werden, die unter den Reaktionsbedingungen inert sind. Zum Beispiel seien genannt : Chlorbenzol, o-, m-, p-Dichlorbenzol, Trichlorbenzol, Nitrobenzol, 1,1,1-Trichlorethan und/oder Tetrachlorkohlenstoff. Es ist jedoch auch möglich, 2,4,6-Trichloranilin selbst als Lösungsmittel in das erfindungsgemäße Verfahren einzusetzen.

Die Menge des inerten organischen Lösungs- und/oder Verdünnungsmittels ist nicht kritisch und kann in weiten Bereichen schwanken. Es kann sowohl in hoher Verdünnung als auch in hohen Konzentrationen gearbeitet werden. Im allgemeinen werden etwa 0,1 bis 50 Teile, bevorzugt 1 bis 10 Teile, an inertem organischem Lösungs- und/oder Verdünnungsmittel auf einen Teil eingesetztes Anilin verwendet.

Das erfindungsgemäße Verfahren kann in der Weise durchgeführt werden, daß man das Ausgangsprodukt in inertes organisches Lösungs- und/oder Verdünnungsmittel einbringt, das Gemisch auf die gewünschte Reaktionstemperatur aufheizt und dann das Chlorierungsmittel zusetzt. Gegebenenfalls wird das Reaktionsgemisch nach Ende der Reaktion zur Zerstörung der Hydrochloride thermisch nachbehandelt. Hierfür sind Temperaturen von etwa 80 bis 250 °C, bevorzugt 90 bis 150 °C, geeignet. Danach wird das Lösungs- und/oder Verdünnungsmittel abdestilliert und das erhaltene rohe 2,4,6-Trichloranilin durch Destillation oder Sublimation in praktisch reines 2,4,6-Trichloranilin überführt. Es ist natürlich auch möglich, das Reaktionsgemisch gemäß dem Stand der Technik durch Wasserwäsche zu behandeln um eine hydrolytische Spaltung der Hydrochloride zu erzielen. Dies ist jedoch aufwendig und wenig vorteilhaft.

Eine andere Möglichkeit, 2,4,6-Trichloranilin in besonders wirtschaftlicher Weise herzustellen, besteht darin, daß man das inerte organische Lösungs- und/oder Verdünnungsmittel mit nur einem Teil des Ausgangsprodukts (etwa 0,001 bis 40 Gew.-%, bevorzugt 1 bis 20 Gew.-%, bezogen auf die gesamt umzusetzende Menge an Anilinderivat) vorlegt und der Rest des Ausgangsprodukts synchron mit der Gesamtmenge des Chlorierungsmittels zugibt. Selbstverständlich ist es auch möglich, die Gesamtmenge des Ausgangsprodukts synchron mit der Gesamtmenge des Chlorierungsmittels dem vorgelegten inerten organischen Lösungs- und/oder Verdünnungsmittel zuzudosieren, d. h. ohne Vorlage von nur einem Teil des Ausgangsproduktes.

Bei dieser Variante kann bei Temperaturen im Bereich von etwa 20 bis 180 °C, vorteilhaft bei 60 bis 175 °C, ganz besonders bevorzugt bei 80 bis 160 °C, gearbeitet werden.

Das erfindungsgemäße Verfahren kann sowohl kontinuierlich als auch diskontinuierlich durchgeführt werden.

Im Vergleich zu dem Stand der Technik wird nach dem erfindungsgemäßen Verfahren 2,4,6-Trichloranilin in einer besseren Ausbeute, einer höheren Raum/Zeit-Ausbeute sowie in einer besseren Produktqualität erhalten. Dies ist umso überraschender, als man aufgrund des Standes der Technik davon ausgehen mußte, daß bei höheren Reaktionstemperaturen, bei Einsatz von freiem Anilinderivat und durch die thermische Behandlung des Rohgemisches die Produktqualität und die Ausbeute an 2,4,6-Trichloranilin vermindert würden.

Das erfindungsgemäße Verfahren sei anhand der folgenden Beispiele erläutert, ohne es jedoch auf diese Beispiele einzuschränken.

## Vergleichsbeispiel (nach US 2 675 409)

1,0 Mol Anilin werden gemäß dem Beispiel 1 der US-PS 2 675 406 mit Chlor behandelt. Man erhält 176 g rohes 2,4,6-Trichloranilin mit einem Gehalt an reinem 2,4,6-Trichloranilin von 67,8 % (Ausbeute : 60,7 %; bestimmt nach HPLC, High pressure liquid Chromatography). Das rohe 2,4,6-Trichloranilin enthält 0,5 % Trichlorphenol, 1,5 % Tetrachlorphenol, 1,5 % Pentachlorphenol, 4 bis 5 % unbekannte Verbindungen sowie ca. 20 % Polymere.

## Beispiel 1

96 g Anilin werden in 750 ml Chlorbenzol gelöst. Man leitet 40 g HCl-Gas in die Lösung und heizt dann auf 90 °C auf. Unter gutem Rühren werden innerhalb von 6 Stunden 432 g Sulfurylchlorid eingetropft. Es wird 15 Minuten bei 90 °C nachgerührt und dann eine weitere Stunde bei 130 °C gerührt. Man destilliert Chlorbenzol ab und gewinnt 204 g rohes Trichloranilin (86,3 %ig), (HPLC) = 89,5 % der Theorie. Sublimation des Rohproduktes ergibt 176 g 2,4,6-Trichloranilin (97,2 %ig) (HPLC).

## Beispiel 2

Die Reaktion wird bei 110 °C analog Beispiel 1 vorgenommen. Man erhält 192 g rohes Trichloranilin 89,1 %ig = 87,5 % der Theorie.

## Beispiel 3

Die Reaktion wird analog Beispiel 1 vorgenommen. Als Lösungsmittel dient 750 ml o-Dichlorbenzol. Man gewinnt 191 g rohes 2,4,6-Trichloranilin 91,3 %ig = 88,7 % der Theorie. Nach Sublimation erhält man 176 g Trichloranilin mit einem Gehalt von 98,7 %.

Beispiel 4

In einem 2 l Dreihalskolben werden 36 g Anilin in 500 ml Chlorbenzol gelöst. Man leitet 15 g gasförmigen Chlorwasserstoff ein. Dann wird auf 110 °C erwärmt. Innerhalb von 8 Stunden werden synchron 864 g Sulfurylchlorid mit 108 g $h^{-1}$ und 170 g Anilin mit 21,3 g $h^{-1}$ eingetragen. Nach dem Eintragen wird 1 Stunde bei 130 °C gerührt. Dann destilliert man Chlorbenzol im steigenden Wasserstrahlvakuum ab. Man erhält 407 g rohes 2,4,6-Trichloranilin 89,9 %ig = 93,1 % der Theorie. Das Produkt enthält neben Resten an Chlorbenzol, 1 % Tetrachloranilin und 6 % Polymere. Die Ausbeute nach Sublimation an reinem (98,2 %ig) Produkt beträgt 366,2 g.

Beispiel 5

93 g Anilin werden in 850 ml Chlorbenzol gelöst. Man läßt bei Raumtemperatur 40 g HCl-Gas einströmen und heizt dann auf 90 °C. Innerhalb von 8 Stunden werden 231 g Chlor eingeleitet. Dann wird 1 Stunde bei 130 °C gerührt und Chlorbenzol im Wasserstrahlvakuum abdestilliert. Man erhält 209 g rohes 2,4,6-Trichloranilin 81,8 %ig (HPLC) = 87,0 % der Theorie. Die Ausbeute nach Sublimation an reinem Produkt (96 %ig) beträgt 175 g.

Beispiel 6

63,8 g o-Chloranilin werden in 375 ml Chlorbenzol gelöst. Es werden 20 g Salzsäuregas eingeleitet und auf 110 °C erhitzt. Man läßt innerhalb von 6 Stunden 139,5 g Sulfurylchlorid zulaufen. Danach wird 1 Stunde bei 130 °C gerührt, Chlorbenzol abdestilliert, bis bei 20 mbar die Sumpftemperatur 110 °C erreicht. Man gewinnt 100 g 2,4,6-Trichloranilin mit einem Gehalt von 84,3 % = 85,4 % der Theorie.

Beispiel 7

Die Mengen und die Verfahrensweise sind analog Beispiel 5, die Temperatur wurde bei 100 °C gehalten. Man gewinnt 210 g 2,4,6-Trichloranilin 87,4 %ig = 93,4 % der Theorie. Die Ausbeute an reinem Produkt (97,6 %ig) beträgt 186 g.

Beispiel 8

In 500 ml Chlorbenzol werden 37,2 g Anilin gegeben. Dann werden unter Kühlung 17,0 g HCl-Gas eingeleitet. Man heizt auf 100 °C auf. Innerhalb von 8 Stunden werden synchron 462 g Chlor und 148,8 g Anilin zudosiert. Es wird 1 Stunde auf 110 °C aufgeheizt und dann Chlorbenzol abdestilliert bis bei 20 mbar der Sumpf eine Temperatur von 80 °C erreicht. Man erhält 410 g rohes Trichloranilin. Nach Sublimation werden 370,3 g 97,2 %iges reines Produkt erhalten (91,6 % der Theorie).

Beispiel 9

In eine Mischung aus 37,2 g Anilin und 1 500 ml Chlorbenzol werden 17,0 g HCl-Gas eingeleitet. Man erwärmt auf 50 °C. Innerhalb von 8 Stunden werden synchron 497 g Chlor und 148,8 g Anilin zudosiert. Es wird zur thermischen Zerstörung der Hydrochloride auf 130 °C geheizt und bei dieser Temperatur 1 Stunde gerührt. Man destilliert das Chlorbenzol ab bis der Sumpf bei 20 mbar 80 °C erreicht. Man gewinnt 409 g rohes 2,4,6-Trichloranilin 77,2 %ig = 80,3 % der Theorie.

Beispiel 10

7 g Anilin werden in 650 ml Chlorbenzol vorgelegt und auf 100 °C erwärmt. Bei dieser Temperatur werden innerhalb 20 Stunden synchron 552,5 g Chlor und 226 g Anilin zudosiert.

Nach einstündigem Rühren bei 130 °C wird das Chlorbenzol im Vakuum abdestilliert.

Man erhält 480 g Trichloranilin (roh) mit einem Gehalt von 89,1 %, entsprechend 87 % der Theorie.

**Patentansprüche**

1. Verfahren zur Herstellung von 2,4,6-Trichloranilin durch Umsetzung von Anilinen der Formel

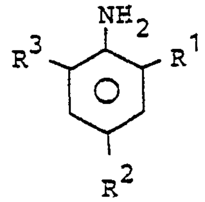

worin $R^1$, $R^2$ und $R^3$ gleich oder verschieden sind und für Wasserstoff oder Halogen stehen und wobei mindestens einer der Reste Wasserstoff bedeutet,
und/oder deren Hydrochloride mit einem Chlorierungsmittel in Gegenwart von inerten, organischen Lösungs- und/oder Verdünnungsmitteln, dadurch gekennzeichnet, daß man die Umsetzung bei Temperaturen oberhalb von 80 °C durchführt.

2. Verfahren nach Anspruch 1, dadurch gekennzeichnet, daß man die Umsetzung bei Temperaturen von 82 bis 180 °C durchführt.

3. Verfahren nach Ansprüchen 1 und 2, dadurch gekennzeichnet, daß man die Umsetzung bei Temperaturen von 85 bis 175 °C durchfürht.

4. Verfahren nach Ansprüchen 1 bis 3, dadurch gekennzeichnet, daß man die Umsetzung bei Temperaturen von 90 bis 170 °C durchführt.

5. Verfahren zur Herstellung von 2,4,6-Trichloranilin durch Umsetzung von Anilinen der Formel

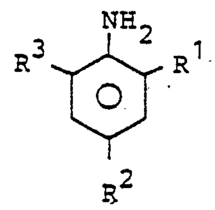

worin $R^1$, $R^2$ und $R^3$ gleich oder verschieden sind und für Wasserstoff oder Halogen stehen und wobei mindestens einer der Reste Wasserstoff bedeutet,
und/oder deren Hydrochloride mit einem Chlorierungsmittel in Gegenwart von inerten, organischen Lösungs- und/oder Verdünnungsmitteln, dadurch gekennzeichnet, daß man das inerte, organische Lösungs- und/oder Verdünnungsmittel mit einem Teil des Ausgangsproduktes vorlegt und den Rest des Ausgangsproduktes synchron mit dem Chlorierungsmittel zugibt.

6. Verfahren nach Anspruch 5, dadurch gekennzeichnet, daß man inertes, organisches Lösungs- und/oder Verdünnungsmittel mit 0,001 bis 40 Gew.-% an Ausgangsprodukt, bezogen auf die gesamt umzusetzende Menge an Anilinderivat, vorlegt und den Rest des Ausgangsprodukts synchron mit der Gesamtmenge des Chlorierungsmittels zugibt.

7. Verfahren nach Ansprüchen 5 und 6, dadurch gekennzeichnet, daß man inertes, organisches Lösungs- und/oder Verdünnungsmittel mit 1 bis 20 Gew.-% an Ausgangsprodukt, bezogen auf die gesamt umzusetzende Menge an Anilinderivat, vorlegt und den Rest des Ausgangsprodukts synchron mit der Gesamtmenge des Chlorierungsmittels zugibt.

8. Verfahren nach Ansprüchen 5 bis 7, dadurch gekennzeichnet, daß man die Umsetzung bei Temperaturen von 20 bis 180 °C durchführt.

9. Verfahren nach Ansprüchen 5 bis 8, dadurch gekennzeichnet, daß man die Umsetzung bei Temperaturen von 60 bis 175 °C durchführt.

**Claims**

1. Process for the preparation of 2,4,6-trichloroaniline by reaction of anilines of the formula

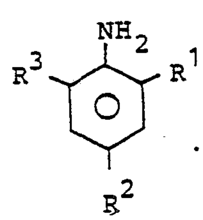

wherein $R^1$, $R^2$ and $R^3$ are identical or different and represent hydrogen or halogen and wherein at least one of the radicals denotes hydrogen,
and/or their hydrochlorides, with a chlorinating agent in the presence of inert organic solvents and/or diluents, characterised in that the reaction is carried out at temperatures above 80 °C.

2. Process according to Claim 1, characterised in that the reaction is carried out at temperatures from 82 to 180 °C.

3. Process according to Claims 1 and 2, characterised in that the reaction is carried out at temperatures from 85 to 175 °C.

4. Process according to Claims 1 to 3, characterised in that the reaction is carried out at temperatures from 90 to 170 °C.

5. Process for the preparation of 2,4,6-trichloroaniline by reaction of anilines of the formula

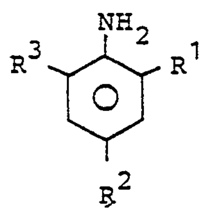

wherein $R^1$, $R^2$ and $R^3$ are identical or different and represent hydrogen or halogen and wherein at least one of the radicals denotes hydrogen,
and/or their hydrochlorides, with a chlorinating agent in the presence of inert organic solvents and/or diluents, characterised in that the inert organic solvent and/or diluent is initially introduced with a part of the starting product, and the rest of the starting product is added synchronously with the chlorinating agent.

6. Process according to Claim 5, characterised in that an inert organic solvent and/or diluent is initially introduced with 0.001 to 40 % by weight of starting product, relative to the total amount of aniline derivative to be reacted, and the rest of the starting product is added synchronously with the total amount of chlorinating agent.

7. Process according to Claims 5 and 6, characterised in that an inert organic solvent and/or diluent is initially introduced with 1 to 20 % by weight of starting product, relative to the total amount of aniline derivative to be reacted, and the rest of the starting product is added synchronously with the total amount of the chlorinating agent.

8. Process according to Claims 5 to 7, characterised in that the reaction is carried out at temperatures from 20 to 180 °C.

9. Process according to Claims 5 to 8, characterised in that the reaction is carried out at temperatures from 60 to 175 °C.

**Revendications**

1. Procédé de fabrication de 2,4,6-trichloraniline par réaction d'anilines de formule :

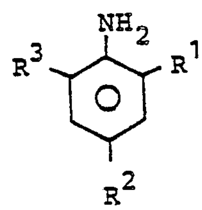

dans laquelle $R^1$, $R^2$ et $R^3$ sont identiques ou différents et représentent de l'hydrogène ou de l'halogène, au moins un des radicaux signifiant de l'hydrogène,
et/ou de ses chlorhydrates, avec un agent de chloruration en présence de solvants et/ou diluants organiques inertes, caractérisé en ce qu'on exécute la réaction à des températures supérieures à 80 °C.

2. Procédé selon la revendication 1, caractérisé en ce qu'on exécute la réaction à des températures de 82 à 180 °C.

3. Procédé selon les revendications 1 et 2, caractérisé en ce qu'on exécute la réaction à des températures de 85 à 175 °C.

4. Procédé selon les revendications 1 à 3, caractérisé en ce qu'on exécute la réaction à des températures de 90 à 170 °C.

5. Procédé de fabrication de 2,4,6-trichloraniline par réaction d'anilines de formule :

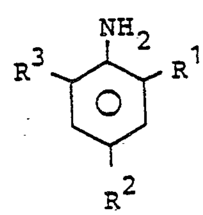

dans laquelle $R^1$, $R^2$ et $R^3$ sont identiques ou différents et représentent de l'hydrogène ou de l'halogène, au moins un des radicaux signifiant de l'hydrogène, et/ou de ses chlorhydrates, avec un agent de chloruration en présence de solvants et/ou diluants organiques inertes, caractérisé en ce qu'on introduit d'avance le solvant et/ou diluant organique inerte avec une partie du produit de départ et l'on ajoute le restant du produit de départ synchroniquement avec l'agent de chloruration.

6. Procédé selon la revendication 5, caractérisé en ce qu'on introduit d'avance le solvant et/ou diluant organique inerte avec 0,001 à 40 % en poids du produit de départ par rapport à la quantité de dérivé d'aniline à faire réagir en tout, et en ce qu'on ajoute le restant du produit de départ synchroniquement avec la quantité totale de l'agent de chloruration.

7. Procédé selon les revendications 5 et 6, caractérisé en ce qu'on introduit d'avance le solvant et/ou diluant organique inerte avec 1 à 20 % en poids du produit de départ par rapport à la quantité de dérivé d'aniline à faire réagir en tout, et en ce qu'on ajoute le restant du produit de départ synchroniquement avec la quantité totale de l'agent de chloruration.

8. Procédé selon les revendications 5 à 7, caractérisé en ce qu'on exécute la réaction à des températures de 20 à 180 °C.

9. Procédé selon les revendications 5 à 8, caractérisé en ce qu'on exécute la réaction à des températures de 60 à 175 °C.